# EUROPEAN PATENT SPECIFICATION

(11) **EP 3 892 220 B1**
(45) Date of publication and mention of the grant of the patent: **23.07.2025**
(21) Application number: 21166943.7
(22) Date of filing: 06.04.2021
(51) Int. Cl.: A61B 18/14, A61B 18/00, A61B 18/12

(54) **BASKET CATHETER WITH SOLID CONDUCTING SPINES AS ELECTRODES FOR IRE**
KORBKATHETER MIT FESTEN LEITENDEN STACHELN ALS ELEKTRODEN FÜR IRE
CATHÉTER À PANIER AYANT DES COLONNES CONDUCTRICES SOLIDES EN TANT QU'ÉLECTRODES POUR IRE

(30) Priority: 07.04.2020 US 202016842648
(43) Date of publication of application: 13.10.2021
(73) Proprietor: Biosense Webster (Israel) Ltd., Yokneam 2066717 (IL)
(72) Inventor: GOVARI, Assaf, Yokneam (IL); ALTMANN, Andres Claudio, Yokneam (IL); BEECKLER, Christopher Thomas, Irvine (US)
(74) Representative: Carpmaels & Ransford LLP

(56) References cited:
- EP-A1- 3 178 429
- WO-A1-2014/124231
- WO-A1-2014/195933
- WO-A2-2015/171921
- US-A1- 2003 233 099

## Description

### FIELD OF THE INVENTION

The present invention relates generally to medical equipment, and particularly to apparatus for ablating tissue within the body.

### BACKGROUND

Irreversible electroporation (IRE) is a soft tissue ablation technique that applies short pulses of strong electrical fields to create permanent and hence lethal nanopores in the cell membrane, thus disrupting the cellular homeostasis (internal physical and chemical conditions). Cell death following IRE results from apoptosis (programmed cell death) and not necrosis (cell injury, which results in the destruction of a cell through the action of its own enzymes) as in other thermal and radiation-based ablation techniques. IRE is commonly used in tumor ablation in regions where precision and conservation of the extracellular matrix, blood flow and nerves are of importance.

International Patent Application Pulication WO2014/195933A1 describes selective cellular ablation by electroporation. In one example, a radially expandable electrode array is pressed into position for electroporating ablation of myocardial tissue by expansion of a frame.

International Patent Application Publication WO2015/171921A2 describes catheter systems and methods for the selective and rapid application of DC voltage to drive irreversible electroporation. In one example, a basket ablation catheter for electroporation energy delivery is described.

### SUMMARY

Embodiments of the present invention that are described hereinbelow provide improved systems for ablation of tissue in the body. The invention is defined in claim 1.

There is therefore provided, in accordance with an embodiment of the present invention, a medical apparatus, which includes a probe. The probe includes an insertion tube configured for insertion into a body cavity of a patient, a basket assembly connected distally to the insertion tube, and a plurality of resilient, conductive spines, which are configured to contact tissue within the body and to allow current to flow between the conductive spines along the entire length of the conductive spines. The apparatus further includes an electrical signal generator configured to apply between one or more pairs of the spines bipolar pulses having an amplitude sufficient to cause irreversible electroporation (IRE) in the tissue contacted by the spines.

In a disclosed embodiment, the spines have respective proximal and distal tips, wherein the proximal tips of the spines are joined mechanically at a proximal end of the basket assembly, and the distal tips of the spines are joined mechanically at a distal end of the basket assembly, and the spines bow radially outward when the basket assembly is deployed in the body cavity, thereby contacting the tissue in the body cavity.

In a further disclosed embodiment, the basket assembly has a stable collapsed state, and the apparatus includes a puller attached to the distal end of the basket assembly and slidably disposed within the insertion tube, so that the spines bow radially outward in response to pulling the puller in a proximal direction through the insertion tube. Additionally or alternatively, the puller includes a reinforced polymer tube.

In some embodiments, the insertion tube includes a flexible catheter configured for insertion into a chamber of a heart of the patient, and the spines are configured to contact and apply the electrical signals to myocardial tissue within the chamber.

In additional embodiments, the spines include drawn metal ribbons, which may be fabricated from a nickel-titanium alloy. Additionally or alternatively, the spines include flexible printed circuit boards bonded to structural members.

In disclosed embodiments, the bipolar pulses applied by the electrical signal generator include a sequence of bipolar pulses having an amplitude of at least 200 V, and a duration of each of the bipolar pulses is less than 20 µs. Additionally or alternatively, the sequence of the bipolar pulses includes pairs of pulses, wherein each pair includes a positive pulse and a negative pulse.

In some embodiments, the electrical signal generator is configured to apply the bipolar pulses between first and second sets of the spines, wherein at least one of the sets includes two or more of the spines.

In further embodiments, the apparatus includes a controller configured to transmit control signals to the electrical signal generator, wherein the electrical signal generator includes a pulse generation assembly, configured to receive the control signals from the controller and to transmit sequences of bipolar pulses with an amplitude and duration responsive to the control signals. The electrical signal generator further includes a pulse routing assembly, which includes a configurable network of switches, which are configured to receive the control signals from the controller, to receive the sequences of bipolar pulses from the pulse generation assembly, and to select sets of the spines responsively to the received control signals, each set including one or more of the spines, so as to the transmit the sequences of bipolar pulses through the selected sets.

The present invention will be more fully understood from the following detailed description of the embodiments thereof, taken together with the drawings in which:

### BRIEF DESCRIPTION OF THE DRAWINGS

Fig. 1 is a schematic pictorial illustration of a system used in an IRE ablation procedure, in accordance with embodiments of the invention;
Fig. 2a is a schematic side view of a basket assembly, in accordance with an embodiment of the invention;
Figs. 2b and 2c are schematic sectional views of spines in a basket assembly, in accordance with two embodiments of the invention;
Figs. 3a and 3b are schematic side views of a basket assembly in its collapsed and expanded states, respectively, in accordance with an alternative embodiment of the invention;
Fig. 4 is a schematic illustration of a bipolar IRE pulse, in accordance with an embodiment of the invention;
Fig. 5 is a schematic illustration of a burst of bipolar pulses, in accordance with an embodiment of the invention; and
Fig. 6 is a block diagram that schematically illustrates an IRE module, in accordance with an embodiment of the invention.

### DETAILED DESCRIPTION OF EMBODIMENTS

### OVERVIEW

IRE is a predominantly non-thermal process, which causes an increase of the tissue temperature by, at most, a few degrees for a few seconds. It thus differs from RF (radio frequency) ablation, which raises the tissue temperature by between 20 and 70°C and destroys cells through heating. IRE utilizes bipolar pulses, i.e., combinations of positive and negative pulses, in order to avoid muscle contraction from a DC voltage. The pulses are applied, for example, between two bipolar electrodes of a catheter. Typically the electrodes are relatively small, for achieving a localized IRE in the tissue. However, the use of small electrodes reduces the size of the electroporated area.

The embodiments of the present invention that are described herein address the problem of increasing the area of electroporation by providing a basket-type catheter with conductive spines, which contact tissue in a body cavity, such as myocardial tissue in the heart. Each spine acts as an electrode, and an electrical signal generator applies bipolar pulses between one or more pairs of the spines with an amplitude sufficient to cause IRE in the tissue contacted by the spines. The pairs of spines through which the pulses are applied are selected according to the region to be electroporated. Using complete spines as electrodes in this manner allows relatively large areas to be electroporated simultaneously.

Additionally or alternatively, a set of multiple spines may be electrically connected together to create a larger "virtual electrode". IRE signals may be applied between one or more pairs of these "virtual electrodes" or sets.

Catheters having an expandable basket assembly in accordance with embodiments of the invention are typically inserted into the heart through a narrow sheath, with the basket assembly in a collapsed state. For deployment in the body cavity, the basket assembly emerges out of the sheath and expands into its operational state. In some embodiments, the spines are made of a resilient material, which is fabricated so as to curve outward into the form of the basket when released from the sheath. The catheter may include a pusher tube within the insertion tube of the catheter. This pusher tube can be pushed distally at the conclusion of the ablation procedure in order to straighten the spines and thus collapse the basket assembly so that it may be pulled back into the sheath, thus facilitating the removal of the catheter from the subject.

In other embodiments, the spines are fabricated so that the basket assembly is stable in a collapsed state rather than in an expanded state. A puller passing through the insertion tube is connected to the distal end of the basket assembly. After the basket assembly emerges from the sheath within a body cavity, pulling on the puller expands the assembly from its stable collapsed state to an expanded state and thus enables its deployment. By releasing the puller, the basket assembly reverts back to its collapsed state, and enables it to be pulled back into the insertion tube for the extraction of the catheter from the subject. This configuration is advantageous in that the puller is typically thinner and more flexible than a pusher tube, thus enabling the catheter to be made thinner and more flexible. The puller may comprise, for example, either a simple wire or alternatively a more complex assembly, such as a polymer tube with braiding or embedded wires to minimize its elongation.

### SYSTEM DESCRIPTION

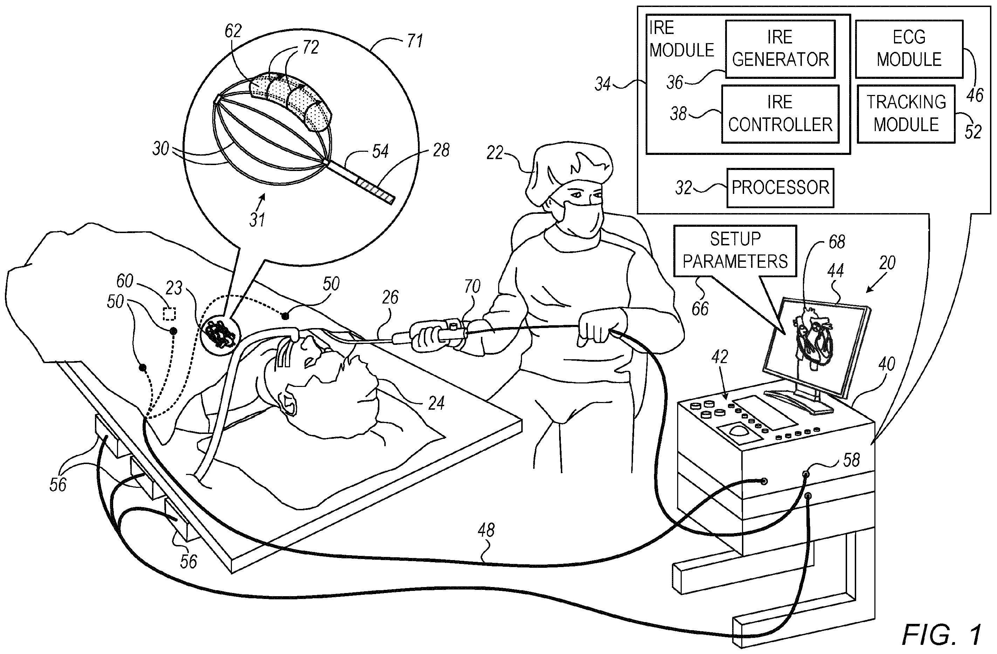

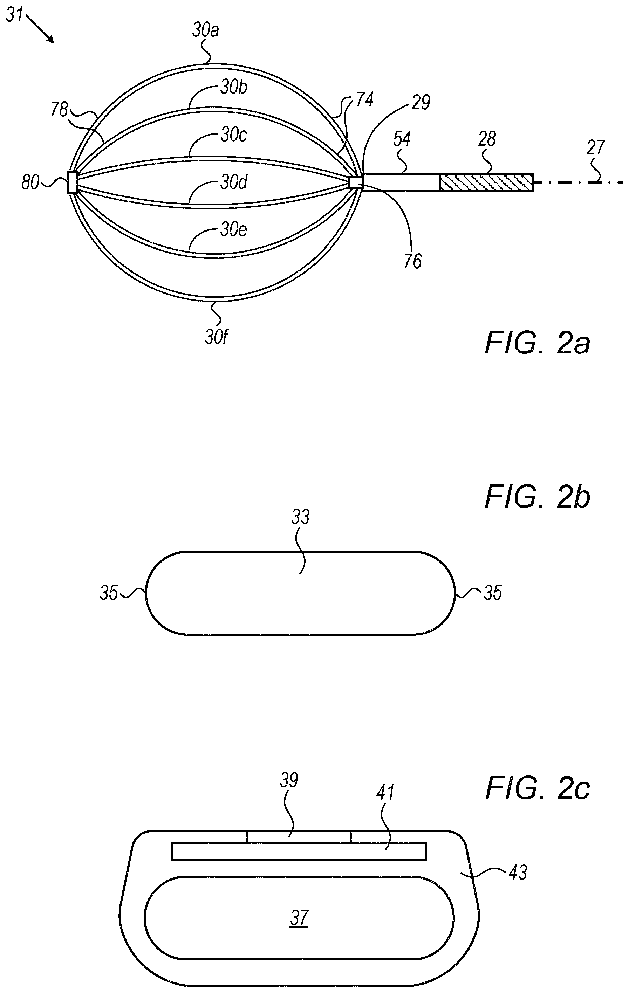

Fig. 1 is a schematic pictorial illustration of a system 20 used in an IRE ablation procedure, in accordance with embodiments of the invention. (In the following description, the IRE ablation procedure will also be referred to as "IRE ablation" or "IRE procedure.") In the pictured embodiment, a physician 22 is performing an IRE ablation procedure in a heart 23 of subject 24 using system 20. Physician 22 is performing the procedure using an ablation catheter 26 comprising an insertion tube 28 with a longitudinal axis 27, wherein a distal end 29 of the insertion tube is connected to a basket assembly 31 comprising multiple conductive spines 30 (shown in more detail in Figs. 2a, 2b and 2c).

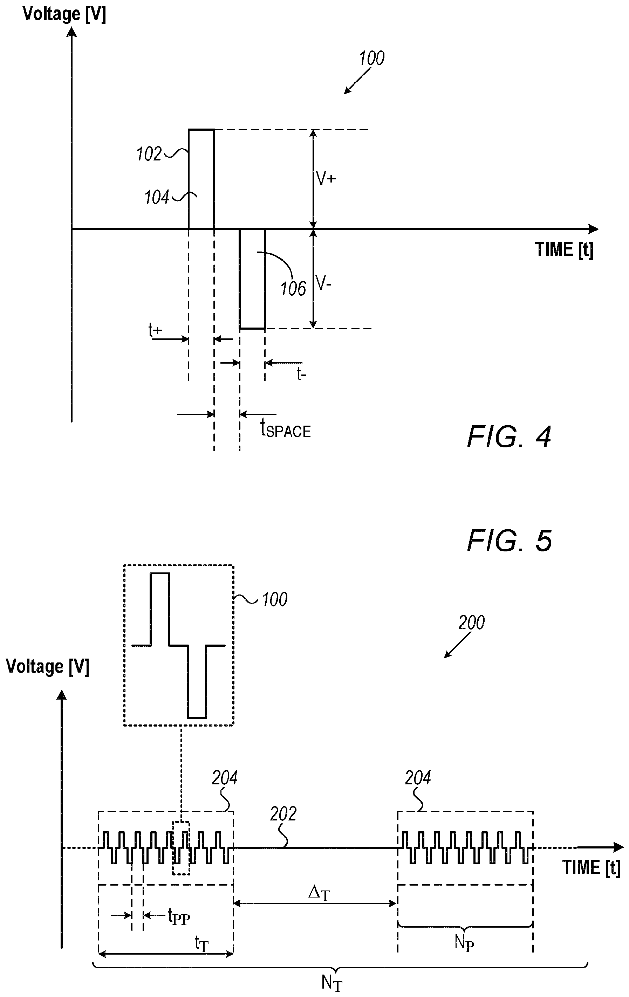

IRE system 20 comprises a processor 32 and an IRE module 34. IRE module 34 comprises an IRE generator 36 and an IRE controller 38, wherein the IRE generator comprises a dedicated pulse generator, as described further hereinbelow. IRE generator 36 generates, under the control of IRE controller 38, IRE signals, which comprise trains of bipolar electrical pulses. The pulses are directed to selected spines 30, which serve as electrodes for performing an IRE procedure. Processor 32 handles the input and output interfaces between IRE system 20 and physician 22, as well as the communication to IRE controller 38. The bipolar electrical pulses and IRE module 34 are further described below with reference to Figs.4 and 5.

Processor 32 and IRE controller 38 each typically comprises a programmable processor, which is programmed in software and/or firmware to carry out the functions that are described herein. Alternatively or additionally, each of them may comprise hard-wired and/or programmable hardware logic circuits, which carry out at least some of these functions. IRE generator 36 comprises analog and digital components and assemblies, for generating the IRE signals that are directed to spines 30. Although processor 32, IRE generator 36, and IRE controller 38 are shown in the figures, for the sake of simplicity, as separate, monolithic functional blocks, in practice some of these functions may be combined in a single processing and control unit.

Processor 32 and IRE module 34 typically reside within a console 40. Console 40 comprises input devices 42, such as a keyboard and a mouse. A display screen 44 is located in proximity to (or integral to) console 40. Display screen 44 may optionally comprise a touch screen, thus providing another input device.

IRE system 20 may additionally comprise one or both of the following modules (typically residing within console 40), connected to suitable interfaces and devices in system 20:
- An electrocardiogram (ECG) module 46 is coupled through a cable 48 to ECG electrodes 50, which are attached to subject 24. ECG module 46 is configured to measure the electrical activity of heart 23.
- A tracking module 52 is coupled to one or more electromagnetic position sensors 54 in the distal end of insertion tube 28, and possibly within basket assembly 31, as well. In the presence of an external magnetic field generated by one or more magnetic-field generators 56, electromagnetic position sensors 54 output signals that vary with the positions of the sensors. Based on these signals, tracking module 52 tracks the positions of spines 30 in heart 23.

The above modules 46 and 52 typically comprise both analog and digital components, and are configured to receive analog signals and transmit digital signals. Each module may additionally comprise hard-wired and/or programmable hardware logic circuits, which carry out at least some of the functions of the module.

Catheter 26 is coupled to console 40 via an electrical interface 58, such as a port or socket. IRE signals are thus carried from IRE generator 36 via interface 58 and wiring inside insertion tube 28 to spines 30 in basket assembly 31. Similarly, signals for tracking the position and orientation of distal end 28 may be received by tracking module 52 via interface 58.

An external electrode 60, or "return patch," may be additionally coupled externally between subject 24, typically on the skin of the subject's torso, and IRE module 34. External electrode 60 may be used for coupling IRE signals between one of spines 30 and the external electrode, thus achieving electroporation that is localized deeper in tissue 62.

Processor 32 receives from physician 22 (or from another user), prior to and/or during the IRE procedure, setup parameters 66 for the procedure. Using one or more suitable input devices 42, physician 22 sets setup parameters 66, selecting one or more pairs of spines 30 to serve as electrodes for activation (for receiving the IRE signals) and the order in which the electrodes are activated, as well as defining the characteristics (timing and amplitude) of the IRE signals. Additionally, processor 32 may display setup parameters 66 on display screen 26. As used herein the pairs of spines are not limited to adjacent spines but in all possible configurations that allow for delivery of biphasic energy between (a) two singular spines acting as two separate electrodes or (b) between two groups of multiple spines. For case (a) one example of two singular spines acting as an electrode "pair" in (a) is spine 30a in Fig. 2 adjacent with spine 30b to define a "pair of electrodes" when biphasic voltage is delivered to these spines 30a and 30b. Alternatively, spine 30a can be paired with a non-neighobring spine 30c as "another pair"; spine 30a with non-neighboring spine 30d as yet another "pair"; spine 30a with distant spine 30f as a further "pair"; spine 30b with distant spine 30d as yet a "pair". In a further variation, spines 30a and 30c can be energized as one an "electrode pair" with spines 30b and 30d energized as a different "electrode pair" and so on in various permutations. For case (b) relating to a group of spines acting as one electrode in concert with another group of different spines acting as another electrode to arrive at a "pair of electrodes", two or more spines (e.g., 30a and 30b) can act as one electrode to operate with two or more spines (30c and 30d) grouped together as another electrode define an electrode "pair" for delivery of biphasic energy to the electrodes (i.e., spines 30a and 30b as one electrode and spines 30c and 30d as the other electrode to define an "electrode pair). Various permutations of single spines acting as a pair of electrodes can be combined with groups of spines acting as electrode pairs can be utilized and are considered to be within the scope of the present invention. For example, the spines of shown in Fig. 2 can be utilized to define two electrode pairs: spines 30a and 30b define one electrode pair, a group of spines 30c+30d (one electrode) and another group of spines 30e+30f (as another electrode) to define the second electrode pair in Fig. 2 (i.e., 30a+30b as the first pair of electrodes; 30c and 30d + 30e and 30f as the second pair of electrodes).

In some embodiments, processor 32 displays on display 44, based on signals received from tracking module 60, a relevant image 68 of the subject's anatomy, such as a map of a chamber of heart 23, which is annotated, for example, to show the current position and orientation of basket assembly 31. Alternatively or additionally, based on signals received from ECG module 46, processor 32 may display on display screen 44 the electrical activity of heart 23.

To begin the procedure, physician 22 inserts catheter 26 into subject 24, for example through the subject's vascular system, and then navigates insertion tube 28, using a control handle 70, to an appropriate site within, or external to, heart 23. At the insertion and navigation stage, basket assembly 31 is in a collapsed form, generally inside a sheath (not shown), in order to provide for an easy insertion into subject 24.

Once insertion tube 28 is positioned in the required area within heart 23, basket assembly 31 is advanced from the sheath, assuming an expanded form. A further detailed description of basket assembly 31 is given in Fig. 2, below. As shown schematically in an inset 71, physician 22 now brings basket assembly 31 into contact with tissue 62 of heart 23, such as myocardial or epicardial tissue. Next, IRE generator 36, under the control of IRE controller 38, generates IRE signals comprising trains of pulses (shown in detail in Fig. 5). The IRE signals are carried through catheter 26, over different respective electrical conductors (not shown), to pairs of spines 30, such that currents 72 generated by the IRE signals flow between the spines in each pair (bipolar ablation), and perform the desired irreversible electroporation of tissue 62 over the extended area between the spines.

Fig. 2a is a schematic side view of basket assembly 31, in accordance with an embodiment of the invention. Basket assembly 31 comprises spines 30, which are in Fig. 2 labelled individually as 30a, 30b, 30c, 30d, 30e, and 30f. Spines 30a-30f comprise long segments of a resilient material, which is conductive or has a conductive coating or a conductive member attached to it. For example, spines 30a-30f may comprise a nickel-titanium alloy, known as nitinol.

Proximal tips 74 of spines 30a-30f are joined mechanically at proximal end 76 of basket assembly 31, and distal tips 78 of the spines are joined mechanically at a distal end 80 of the basket assembly. (Proximal and distal tips 74 and 78, respectively, of spines 30a-30f, however, are insulated electrically from one another so that the spines can serve as separate electrodes.) Spines 30a-30f are fabricated so that basket assembly 31 has an expanded state as its stable state. Thus, spines 30a-30f bow radially outward when the basket assembly is deployed in a body cavity, thereby contacting tissue 62 in the body cavity. Spines 30a-30f are electrically coupled via conductors within catheter 26 to IRE generator 36 (Fig. 1) for receiving IRE ablation signals.

The IRE signals received from IRE generator 36 are coupled, for example, between spines 30a and 30b, causing the electroporation to take place between these two spines. Due to the applied IRE signals, currents 72 (Fig. 1) flow between spines 30a and 30b along their entire length, thus causing electroporation over a large area in tissue 62. For example, applying basket assembly 31 to pulmonary vein ablation will cause electroporation over a 6-12 mm wide ring around the pulmonary vein.

The IRE signals may be coupled between any pair of spines 30a-30f, although typically the signals will be applied between pairs of adjacent spines. Additionally or alternatively, the signals may be applied simultaneously or in alternation between several pairs of spines. Although basket assembly 31 is depicted in Fig. 2 to comprise six spines, other numbers of spines, both smaller and larger than six, may be used. Additionally or alternatively, a set of multiple spines 30 may be electrically connected together to form a larger "virtual electrode" comprising multiple spines. IRE signals may be applied between one or more pairs of these "virtual electrodes" or sets.

Figs. 2b and 2c are schematic sectional views of spines 30, in accordance with two embodiments of the invention.

Fig. 2b is a schematic sectional view of spine 30, wherein the spine comprises a drawn nitinol ribbon 33. Nitinol ribbon 33 serves both as a structural member of spine 30 and as an electrical conductor for currents 72 (Fig. 1). Forming nitinol ribbon 33 by the process of drawing is advantageous in that resulting edges 35 of the ribbon are rounded (as opposed to sharp edges formed in a ribbon cut from a sheet of nitinol), thus avoiding damage to tissue 62 touched by spines 30.

Fig. 2c is a schematic sectional view of spine 30 in an alternative embodiment, wherein spine 30 comprises a composite of a structural member 37 and a conductive member 39. Structural member 37 comprises, for example, as in Fig. 2b, a drawn nitinol ribbon. Conductive member 39 comprises a conductive film, such as gold, which is disposed on a flexible printed circuit board (PCB) 41, which in turn is bonded to structural member 37 with suitable bonding and insulating materials 43, such as a biocompatible epoxy or other adhesive. Thus, structural member 37 is insulated from tissue 62, and currents 72 are conducted to the tissue by conductive member 39, which is exposed to the tissue.

Alternatively, spine 30 may comprise a conductive or non-conductive structural member with a conductive coating (not shown).

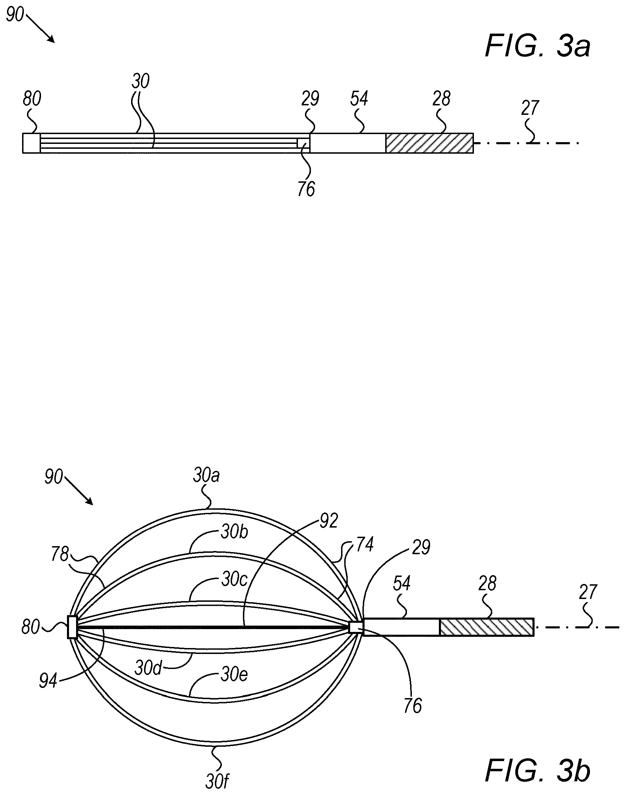

Figs. 3a and 3b are schematic side views of a basket assembly 90 in its collapsed and expanded states, respectively, in accordance with an alternative embodiment of the invention. The same numerical labels as in Figs. 1 and 2 are used for similar items in Figs. 3a and 3b. As in Figs. 2a, 3a, and 3b, spines 30 comprise long segments of a resilient material, which is conductive, or has a conductive coating or a conductive member attached to it.

Fig. 3a shows basket assembly 90 in its collapsed state, in which spines 30 are straight and aligned parallel to longitudinal axis 27. This is the stable state of basket assembly 90: When it is pushed out of the sheath, assembly 90 remains in the collapsed state, unless forced into an expanded state. Ablation catheter 26 contains a puller 92, which extends through and is able to move longitudinally within insertion tube 28. Puller 92 has a distal end 94 attached to distal end 80 of basket assembly 90, and a proximal end (not shown) attached to control handle 70. Since proximal ends 74 of spines 30 are secured to insertion tube 28, pulling puller 92 from its proximal end shortens the distance between the respective distal and proximal ends 78 and 74 of spines 30, and thereby causes the spines to bow outward for deployment, as shown in Fig. 3b. Releasing puller 92 allows basket assembly 90 to collapse back to its stable state (Fig. 3a), due to the resilience of spines 30, for extracting catheter 26 from subject 24.

Puller 92 may comprise any suitable elongated component having sufficient tensile strength and bending flexibility. For example, puller 92 may comprise a simple wire. Alternatively, puller 92 may comprise a more complex structure, such as a polymer tube with reinforcement in the form of braiding or embedded wires to minimize its elongation.

Fig. 5 is a schematic illustration of a bipolar IRE pulse 100, in accordance with an embodiment of the invention.

A curve 102 depicts the voltage V of bipolar IRE pulse 100 as a function of time t in an IRE ablation procedure. Bipolar IRE pulse 100 comprises a positive pulse 104 and a negative pulse 106, wherein the terms "positive" and "negative" refer to an arbitrarily chosen polarity of the two spines 30 between which the bipolar pulse is applied. The amplitude of positive pulse 104 is labeled as V+, and the temporal width of the pulse is labeled as t+. Similarly, the amplitude of negative pulse 106 is labeled as V-, and the temporal width of the pulse is labeled as t-. The temporal spacing between positive pulse 104 and negative pulse 106 is labeled as t_{SPACE}. Typical values for the parameters of bipolar pulse 100 are given in Table 1, below.

Fig. 5 is a schematic illustration of a burst 200 of bipolar pulses, in accordance with an embodiment of the invention.

In an IRE procedure, the IRE signals are delivered to spines 30 as one or more bursts 200, depicted by a curve 202. Burst 200 comprises N_{T} pulse trains 204, wherein each train comprises N_{P} bipolar pulses 100. The length of pulse train 204 is labeled as t_{T}. The period of bipolar pulses 100 within a pulse train 204 is labeled as t_{PP}, and the interval between consecutive trains is labeled as Δ_{T}, during which the signals are not applied. Typical values for the parameters of burst 200 are given in Table 1, below.

**Table 1: Typical values for the parameters of IRE signals**

| Parameter | Symbol | Typical values |
|---|---|---|
| Pulse amplitudes | V+, V- | 200-2000 V |
| Pulse widths | t+, t- | 0.5-5 µs |
| Spacing between positive and negative pulse | t_{SPACE} | 0.1-5 µs |
| Period of bipolar pulses in a pulse train | t_{PP} | 1-20 µs |
| Length of pulse train | t_{T} | 5-100 µs |
| Number of bipolar pulses in a pulse train | N_{P} | 1-100 |
| Spacing between consecutive pulse trains | Δ_{T} | 0.3-1000 ms |
| Number of pulse trains in a burst | N_{T} | 1-100 |
| Length of a burst | | 0-500 ms |
| Energy per channel | | ≤60 J |
| Total time for IRE signal delivery | | ≤10 s |

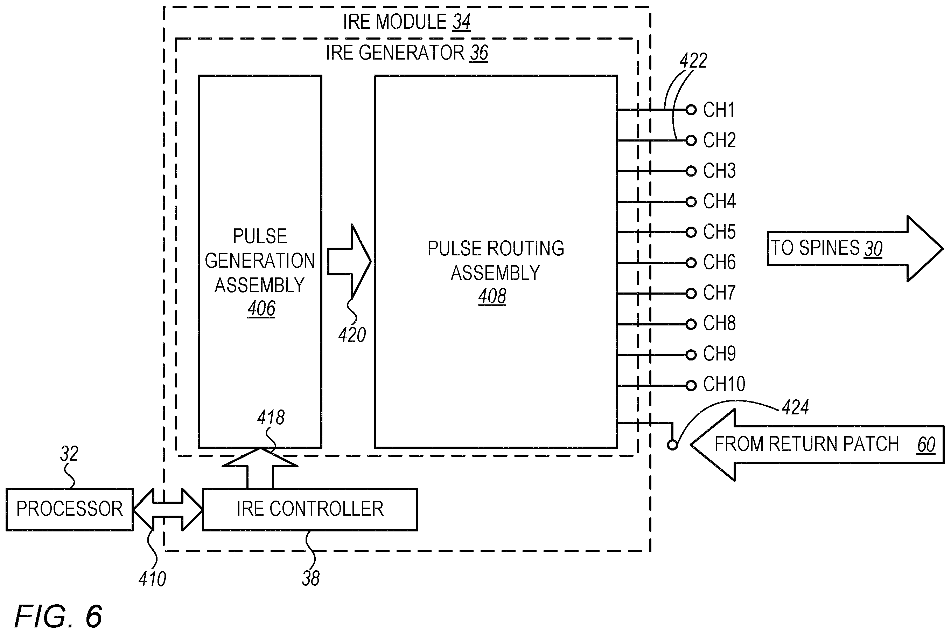

Fig. 6 is a block diagram that schematically shows details of IRE module 34, in accordance with an embodiment of the invention. As explained above in regard to Fig. 1, IRE module 34 comprises IRE generator 36 and IRE controller 38. IRE generator 36 comprises a pulse generation assembly 406 and a pulse routing assembly 408. Pulse generation assembly 406 is configured to receive control signals from IRE controller 38 (as described below) and to transmit sequences of bipolar pulses with an amplitude and duration responsive to the control signals. Pulse routing assembly 408 comprises a configurable network of switches, which are configured to receive control signals from IRE controller 38, to receive sequences of bipolar pulses from pulse generation assembly 405, and to select pairs of spines 30 responsively to the received control signals so as to the transmit the sequences of bipolar pulses through the selected pairs.

IRE controller 38 communicates with processor 32 through bi-directional signals 410, wherein the processor communicates to the IRE controller commands reflecting setup parameters 66. IRE controller 38 further communicates to pulse generation assembly 406 digital command signals 418, derived from setup parameters 66, commanding IRE generator 36 to generate IRE pulses, such as those shown in Fig. 4, above. These IRE pulses are sent to pulse routing assembly 408 as analog pulse signals 420, as directed by IRE controller 38.

Pulse routing assembly 408 is coupled to spines 30 through output channels 422, as well as (optionally) to return patch 60 through a connection 424. For example, when pulse routing assembly 408 is coupled to six spines 30a-30f of basket assembly 31 (Fig. 2), six of output channels 422 are coupled to the spines. Pulse routing assembly 408 is driven by IRE controller 38 to couple the IRE pulses into spines 30 as defined by setup parameters 66. Specifically, IRE controller 38 drives pulse routing assembly 408 to couple the IRE pulses to one or more selected pairs of spines 30. Routing assembly 408 may also electrically couple multiple spines 30 together to form a set that can serve as a "virtual electrode". Thus IRE controller 38 controls both the generation and the routing of the IRE pulses into spines 30.

Although Fig. 6 shows ten channels 422, IRE generator 36 may alternatively comprise a different number of channels, for example 8, 16, or 20 channels, or any other suitable number of channels.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are within the scope of the claims.

## Claims

1. A medical apparatus, comprising:
a probe, comprising:
an insertion tube (28) configured for insertion into a body cavity of a patient; and
a basket assembly (31) connected distally to the insertion tube (28) and comprising a plurality of conductive spines (30), which are configured to contact tissue within the body and to allow current to flow between the conductive spines (30) along the entire length of the conductive spines (30); and
an electrical signal generator (36) configured to apply between one or more pairs of the spines (30) bipolar pulses having an amplitude sufficient to cause irreversible electroporation (IRE) in the tissue contacted by the spines (30).

2. The apparatus according to claim 1, wherein the spines (30) have respective proximal and distal tips, wherein the proximal tips of the spines (30) are joined mechanically at a proximal end of the basket assembly (31), and the distal tips of the spines (30) are joined mechanically at a distal end of the basket assembly (31), and the spines (30) bow radially outward when the basket assembly (31) is deployed in the body cavity, thereby contacting the tissue in the body cavity.

3. The apparatus according to claim 2, wherein the basket assembly (31) has a stable collapsed state, and wherein the apparatus comprises a puller attached to the distal end of the basket assembly (31) and slidably disposed within the insertion tube (28), so that the spines (30) bow radially outward in response to pulling the puller in a proximal direction through the insertion tube (28).

4. The apparatus according to claim 3, wherein the puller comprises a reinforced polymer tube.

5. The apparatus according to claim 1, wherein the insertion tube (28) comprises a catheter configured for insertion into a chamber of a heart of the patient, and the spines (30) are configured to contact and apply the electrical signals to myocardial tissue within the chamber.

6. The apparatus according to claim 1, wherein the spines (30) comprise drawn metal ribbons.

7. The apparatus according to claim 6, wherein the metal ribbons comprise a nickel-titanium alloy.

8. The apparatus according to claim 1, wherein the spines (30) comprise flexible printed circuit boards bonded to structural members.

9. The apparatus according to claim 1, wherein the bipolar pulses applied by the electrical signal generator (36) comprise a sequence of bipolar pulses having an amplitude of at least 200 V, and a duration of each of the bipolar pulses is less than 20 µs.

10. The apparatus according to claim 9, wherein the sequence of the bipolar pulses comprises pairs of pulses, wherein each pair comprises a positive pulse and a negative pulse.

11. The apparatus according to claim 1, wherein the electrical signal generator (36) is configured to apply the bipolar pulses between first and second sets of the spines (30), wherein at least one of the sets comprises two or more of the spines (30).

12. The apparatus according to claim 1, and comprising a controller (38) configured to transmit control signals to the electrical signal generator (36), wherein the electrical signal generator (36) comprises:
a pulse generation assembly (406), configured to receive the control signals from the controller and to transmit sequences of bipolar pulses with an amplitude and duration responsive to the control signals; and
a pulse routing assembly (408), comprising a configurable network of switches, which are configured to receive the control signals from the controller, to receive the sequences of bipolar pulses from the pulse generation assembly (406), and to select sets of the spines (30) responsively to the received control signals, each set comprising one or more of the spines (30), so as to the transmit the sequences of bipolar pulses through the selected sets.

## Patentansprüche

1. Medizinische Einrichtung, umfassend:
eine Sonde, umfassend:
einen Einführschlauch (28), der für eine Einführung in eine Körperhöhle eines Patienten konfiguriert ist; und
eine Korbanordnung (31), die mit dem Einführschlauch (28) distal verbunden ist und umfassend eine Vielzahl von leitfähigen Graten (30), die konfiguriert sind, um Gewebe innerhalb des Körpers zu berühren und um Strom zu ermöglichen, zwischen den leitfähigen Graten (30) entlang der gesamten Länge der leitfähigen Grate (30) zu fließen; und
einen Generator (36) elektrischer Signale, der konfiguriert ist, um zwischen einem oder mehreren Paaren der Grate (30) bipolare Impulse anzulegen, die eine Amplitude aufweisen, die ausreicht, um eine irreversible Elektroporation (IRE) in dem Gewebe zu verursachen, das durch die Grate (30) berührt wird.

2. Einrichtung nach Anspruch 1, wobei die Grate (30) jeweilige proximale und distale Spitzen aufweisen, wobei die proximalen Spitzen der Grate (30) an einem proximalen Ende der Korbanordnung (31) verknüpft sind und die distalen Spitzen der Grate (30) an einem distalen Ende der Korbanordnung (31) verknüpft sind und sich die Grate (30) radial nach außen biegen, wenn die Korbanordnung (31) in der Körperhöhle eingesetzt wird, wobei sie dadurch das Gewebe in der Körperhöhle berühren.

3. Einrichtung nach Anspruch 2, wobei die Korbanordnung (31) einen stabilen zusammengeklappten Zustand aufweist und wobei die Einrichtung eine Herausziehvorrichtung umfasst, die an dem distalen Ende der Korbanordnung (31) befestigt ist und verschiebbar innerhalb des Einführschlauchs (28) angeordnet ist, so dass sich die Grate (30) als Reaktion auf ein Herausziehen der Herausziehvorrichtung in eine proximale Richtung über den Einführschlauch (28) radial nach außen biegen.

4. Einrichtung nach Anspruch 3, wobei die Herausziehvorrichtung einen verstärkten Polymerschlauch umfasst.

5. Einrichtung nach Anspruch 1, wobei der Einführschlauch (28) einen Katheter umfasst, der für die Einführung in eine Kammer eines Herzens des Patienten konfiguriert ist, und die Grate (30) konfiguriert sind, um das Myokardgewebe innerhalb der Kammer zu berühren und die elektrischen Signale an dieses anzulegen.

6. Einrichtung nach Anspruch 1, wobei die Grate (30) gezogene Metallstreifen umfassen.

7. Einrichtung nach Anspruch 6, wobei die Metallstreifen eine Nickel-TitanLegierung umfassen.

8. Einrichtung nach Anspruch 1, wobei die Grate (30) flexible Leiterplatten umfassen, die mit Strukturelementen gebunden sind.

9. Einrichtung nach Anspruch 1, wobei die bipolaren Impulse, die durch den Generator (36) elektrischer Signale angelegt werden, eine Folge bipolarer Impulse umfasst, die eine Amplitude von mindestens 200 V aufweisen und eine Dauer jedes der bipolaren Impulse weniger als 20 µs beträgt.

10. Einrichtung nach Anspruch 9, wobei die Folge der bipolaren Impulse Impulspaare umfasst, wobei jedes Paar einen positiven Impuls und einen negativen Impuls umfasst.

11. Einrichtung nach Anspruch 1, wobei der Generator (36) elektrischer Signale konfiguriert ist, um die bipolaren Impulse zwischen ersten und zweiten Sätzen der Grate (30) anzulegen, wobei mindestens einer der Sätze zwei oder mehr Grate (30) umfasst.

12. Einrichtung nach Anspruch 1, und umfassend eine Steuervorrichtung (38), die konfiguriert ist, um Steuersignale an den Generator (36) elektrischer Signale zu übertragen, wobei der Generator (36) elektrischer Signale umfasst:
eine Impulsgenerierungsanordnung (406), die konfiguriert ist, um die Steuersignale von der Steuervorrichtung zu empfangen und um Folgen bipolarer Impulse mit einer Amplitude und Dauer reagierend auf die Steuersignale zu übertragen, und
eine Impulsrouting-Anordnung (408), umfassend ein konfigurierbares Netzwerk von Schaltern, die konfiguriert sind, um die Steuersignale von der Steuervorrichtung zu empfangen, um die Folgen bipolarer Impulse von der Impulsgenerierungsanordnung (406) zu empfangen und um Sätze der Grate (30) als Reaktion auf die empfangenen Steuersignale auswählen, jeder Satz umfassend einen oder mehrere der Grate (30), um die Folgen bipolarer Impulse über die ausgewählten Sätze zu übertragen.

## Revendications

1. Appareil médical comprenant :
une sonde, comprenant :
un tube d'insertion (28) conçu pour être inséré dans une cavité corporelle d'un patient ; et
un ensemble panier (31) relié distalement au tube d'insertion (28) et comprenant une pluralité d'épines (30) conductrices, qui sont conçues pour entrer en contact avec le tissu à l'intérieur du corps et pour permettre au courant de circuler entre les épines conductrices (30) sur toute la longueur des épines (30) conductrices ; et
un générateur de signaux électriques (36) configuré pour appliquer entre une ou plusieurs paires d'impulsions bipolaires des épines (30) ayant une amplitude suffisante pour provoquer une électroporation irréversible (IRE) dans le tissu en contact avec les épines (30).

2. Appareil selon la revendication 1, dans lequel les épines (30) sont des extrémités proximales et distales respectives, dans lequel les extrémités proximales des épines (30) sont raccordées mécaniquement à une extrémité proximale de l'ensemble panier (31), et les extrémités distales des épines (30) sont raccordées mécaniquement à une extrémité distale de l'ensemble panier (31), et les épines (30) s'inclinent radialement vers l'extérieur lorsque l'ensemble panier (31) est déployé dans la cavité corporelle, ce qui permet d'entrer en contact avec le tissu dans la cavité corporelle ;

3. Appareil selon la revendication 2, dans lequel l'ensemble panier (31) a un état effondré stable, et dans lequel l'appareil comprend un extracteur fixé à l'extrémité distale de l'ensemble panier (31) et disposé de manière coulissante à l'intérieur du tube d'insertion (28), de sorte que les épines (30) s'inclinent radialement vers l'extérieur en réponse à la traction de l'extracteur dans une direction proximale à travers le tube d'insertion (28).

4. Appareil selon la revendication 3, dans lequel l'extracteur comprend un tube en polymère renforcé.

5. Appareil selon la revendication 1, dans lequel le tube d'insertion (28) comprend un cathéter conçu pour être inséré dans une chambre d'un cœur du patient, et les épines (30) sont conçues pour entrer en contact avec le tissu myocardique à l'intérieur de la chambre et y appliquer de l'énergie électrique.

6. Appareil selon la revendication 1, dans lequel les épines (30) comprennent des rubans métalliques tirés.

7. Appareil selon la revendication 6, dans lequel les rubans métalliques comprennent un alliage nickel-titane.

8. Appareil selon la revendication 1, dans lequel les épines (30) comprennent des cartes de circuits imprimés flexibles collées à des éléments structurels.

9. Appareil selon la revendication 1, dans lequel les impulsions bipolaires appliquées par le générateur de signaux électriques (36) comprennent une séquence d'impulsions bipolaires ayant une amplitude d'au moins 200 V et une durée de chacune des impulsions bipolaires inférieure à 20 µs.

10. Appareil selon la revendication 9, dans lequel la séquence des impulsions bipolaires comprend des paires d'impulsions, dans lequel chaque paire comprend une impulsion positive et une impulsion négative.

11. Appareil selon la revendication 1, dans lequel le générateur de signaux électriques (36) est configuré pour appliquer les impulsions bipolaires entre le premier et le second ensemble des épines (30), dans lequel au moins un des ensembles comprend deux ou plusieurs des épines (30).

12. Appareil médical selon la revendication 1, et comprenant un dispositif de commande (38) configuré pour transmettre des signaux de commande au générateur de signaux électriques (36), et dans lequel le générateur de signaux électriques (36) comprend :
un ensemble de génération d'impulsions (406), configuré pour recevoir les signaux de commande du dispositif de commande et pour transmettre des séquences d'impulsions bipolaires avec une amplitude et une durée en réponse aux signaux de commande ; et
un ensemble de routage d'impulsions (408), comprenant un réseau configurable de commutateurs, qui sont configurés pour recevoir les signaux de commande à partir du dispositif de commande, pour recevoir les séquences d'impulsions bipolaires provenant de l'ensemble générateur d'impulsions (406) et pour sélectionner des ensembles des épines (30) sensible aux signaux de commande reçus, chaque ensemble comprenant une ou plusieurs des épines (30), afin de transmettre les séquences d'impulsions bipolaires à travers les ensembles sélectionnés.
